# EUROPEAN PATENT APPLICATION

(11) **EP 2 955 523 A1**
(43) Date of publication of application: **16.12.2015**
(21) Application number: 14305886.5
(22) Date of filing: 12.06.2014
(51) Int. Cl.: G01N 33/68

(54) **A novel soluble biomarker for insulin resistance**

(71) Applicant: SANOFI, 75008 Paris (FR); Universität Leipzig, 04109 Leipzig (DE)
(72) Inventor: The designation of the inventor has not yet been filed
(74) Representative: Löwrick, Oliver

(57) **Abstract**

The present invention relates to a novel soluble biomarker for insulin resistance: 1-methyl nicotinamide (MNA). The invention relates to methods of determining the degree of insulin resistance in a patient. The invention further relates to methods for determining whether a subject with insulin resistance or type 2 diabetes will benefit from a treatment with an inhibitor of nicotinamide-N-methyltransferase (NNMT) or whether a subject responds to a therapeutic treatment with an NNMT inhibitor. The invention also relates to methods of adjusting the dose of an NNMT inhibitor applied for therapeutic treatment of insulin resistance or type-2 diabetes

## Description

### FIELD OF THE INVENTION

The present invention relates to the field of clinical diagnostics, in particular to the diagnosis of insulin resistance.

### BACKGROUND OF THE INVENTION

Increased abdominal adiposity, especially expansion of visceral adipose tissue, has been identified as a major risk factor for the development of insulin resistance, type-2 diabetes (T2D) and cardiovascular disease. How visceral adiposity contributes to the development of T2D is not fully understood, mechanisms that have been proposed include macrophage deposition and activation, release of pro-inflammatory cytokines and free fatty acids that exacerbate systemic insulin resistance and loss of beta-cell function.

Nicotinamide, the amide form of niacin or vitamin B3, is a precursor in the synthesis of NAD⁺ and NADP⁺ that play a role in numerous biological processes like energy production, regulation of cellular redox state, circadian rhythm, and longevity. Prolonged high niacin or nicotinamide intake have been reported to reduce insulin sensitivity in subjects with normal or impaired glucose tolerance. Nicotinamide-N-methyltransferase (NNMT) is the major nicotinamide-metabolizing enzyme but also involved in the detoxification of xenobiotics. Specifically, it catalyzes the transfer of a methyl group from S-adenosylmethionine (SAM) to the ring nitrogen of nicotinamide thus yielding 1-methylnicotinamide (MNA) and S-adenosylhomocysteine (SAH). NNMT is primarily expressed in the liver but also in other organs like lung, muscle, kidney, heart, skin and adipose tissue. In the latter, an increase in NNMT catalytic activity was observed in mice fed a high-fat diet for three months (Riederer et al, Atherosclerosis, 2009, 204:412-417) whereas no change in liver NNMT activity was detected. In contrast, NNMT expression both in adipose tissue and liver was recently found to be up-regulated in mouse-models of obesity and diabetes. Of note, reduction in liver and white adipose tissue NNMT expression by treatment with an antisense oligonucleotide was shown to protect against the development of diet-induced obesity and insulin resistance in a mouse model (Kraus D. et al., Nature 2014 508:258-262). However, whether regulation of NNMT expression is associated with insulin resistance or T2D in humans has so far been unknown.

### TECHNICAL PROBLEMS UNDERLYING THE PRESENT INVENTION

Type 2 diabetes and prediabetes are associated with two deficiencies that may present separately or in combination: (1) the cells in the individual's body are resistant to insulin; i.e. although the pancreas produces a sufficient amount of insulin, glucose cannot enter the individual's cells; and (2) the individual's beta cells in the pancreas do not produce sufficient insulin.

In the prior art, it was difficult to determine whether a patient exhibits insulin resistance. In particular, the degree of insulin resistance could only be determined in a lengthy and expensive clinical procedure, the hyperinsulinemic-euglycemic clamp.

One important finding of the present invention is that the extent of insulin resistance in a subject correlates with NNMT expression in white adipose tissue (WAT). The inventors further found out that NNMT expression in WAT surprisingly correlated with plasma MNA concentrations so that also the extent of insulin resistance in a subject correlates with plasma MNA concentration.

Consequently, through the present invention, subjects with impaired insulin sensitivity can be identified by a simple assay based on a soluble biomarker present in blood. Furthermore, the degree of insulin resistance in a subject can be determined quantitatively by measuring MNA concentrations in blood, thereby avoiding the lengthy and expensive clinical procedure of the prior art, namely hyperinsulinemic-euglycemic clamp.

In addition, the present inventors found out that the novel soluble biomarker MNA can be used for the stratification of patients who may benefit from treatment with an NNMT inhibitor and to monitor the effectiveness of such a treatment with an NNMT inhibitor.

The above overview does not necessarily describe all advantages associated with and problems solved by the present invention.

### SUMMARY OF THE INVENTION

In a first aspect the present invention relates to a method of determining whether a patient with insulin resistance or type-2 diabetes (T2D) will benefit from a therapeutic treatment with an inhibitor of nicotinamide-N-methyltransferase (NNMT), said method comprising the steps:
- determining the level of 1-methylnicotinamide (MNA) in a blood sample isolated from the patient; and
- comparing the level of MNA in the blood sample with a reference level of MNA;
wherein an increased MNA level in the blood sample isolated from the patient as compared to the reference level of MNA indicates that the patient will benefit from said therapeutic treatment.

In a second aspect the present invention relates to a method of determining whether a patient with insulin resistance or type-2 diabetes (T2D) responds to a therapeutic treatment with an inhibitor of nicotinamide-N-methyltransferase (NNMT), said method comprising the steps:
- determining the level of 1-methylnicotinamide (MNA) in a blood sample isolated from the patient; and
- comparing the level of MNA in the blood sample with a pre-treatment level of MNA; wherein a decrease of the MNA level in the blood sample isolated from the patient as compared to the pre-treatment level of MNA indicates that the patient responds to said therapeutic treatment; and
wherein the patient is a patient to whom at least once an inhibitor of NNMT is administered or has been administered.

In a third aspect the present invention relates to a method of adjusting the dose of a therapeutic drug applied for therapeutic treatment of insulin resistance or type-2 diabetes (T2D) in a patient comprising the steps:
- determining the level of 1-methylnicotinamide (MNA) in a blood sample isolated from the patient; and
- comparing the level of MNA in the blood sample with a pre-treatment level of MNA; wherein the patient is a patient to whom at least once an inhibitor of NNMT is administered or has been administered; and
wherein said therapeutic drug is an inhibitor of nicotinamide-N-methyltransferase (NNMT).

In a fourth aspect the present invention relates to a method for determining the degree of insulin resistance in a patient, comprising the steps:
- determining the level of MNA in a blood sample isolated from the patient; and
- comparing the level of MNA in the blood sample with a reference level of MNA;
wherein the degree of the increase of the MNA level in the blood sample isolated from the patient as compared to the reference level of MNA is indicative for the degree of insulin resistance in the patient.

This summary of the invention does not necessarily describe all features of the present invention. Other embodiments will become apparent from a review of the ensuing detailed description.

### BRIEF DESCRIPTION OF THE FIGURES

Figure 1. Association of adipose tissue NNMT expression and T2D. Expression of NNMT in either omental or subcutaneous adipose tissue was significantly higher in people with T2D compared to non-diabetic subjects (females: p=0.001 for omental, p=0.01 for subcutaneous WAT, males: p=0.04 for omental and subcutaneous WAT). Error bars indicate SEM.
Figure 2. Correlation between plasma 1-Methylnicotinamide (MNA) concentrations and NNMT expression in omental adipose tissue.
Fig. 2(A) shows the correlation between plasma MNA concentrations and *NNMT* expression in omental adipose tissue in female, non-diabetic subjects (N=73).
Fig. 2(B) shows the correlation between plasma MNA concentrations and *NNMT* expression in omental adipose tissue in male, non-diabetic subjects (N=38).
Fig. 2(C) shows the correlation between plasma MNA concentrations and *NNMT* expression in omental adipose tissue in female subjects with T2D (N=58).
Fig. 2(D) shows the correlation between plasma MNA concentrations and *NNMT* expression in omental adipose tissue in male subjects with T2D (N=33).
Figure 3: Correlation between plasma 1-Methylnicotinamide (MNA) concentrations and NNMT expression in subcutaneous adipose tissue.
Fig. 3(A) shows the correlation between plasma MNA concentrations and *NNMT* expression in subcutaneous adipose tissue in female, non-diabetic subjects (N=73).
Fig. 3(B) shows the correlation between plasma MNA concentrations and *NNMT* expression in subcutaneous adipose tissue in male, non-diabetic subjects (N=38).
Fig. 3(C) shows the correlation between plasma MNA concentrations and *NNMT* expression in subcutaneous adipose tissue in female subjects with T2D (N=58).
Fig. 3(D) shows the correlation between plasma MNA concentrations and *NNMT* expression in subcutaneous adipose tissue in male subjects with T2D (N=33).
Figure 4. Association of adipose tissue NNMT expression and insulin resistance for the subgroup of patients for which there were clamp data available (N=85).
Fig. 4(A) shows a comparison of *NNMT* expression between individuals with GIR<50 µmol/kg/min and individuals with GIR>50 µmol/kg/min. Expression of *NNMT* in omental (p<0.001) but not subcutaneous (p=0.33) adipose tissue was significantly higher in people with GIR<50 µmol/kg/min.
Fig. 4(B) shows the correlation of GIR and *NNMT expression* in omental WAT in subjects with GIR<50 µmol/kg/min. A negative correlation of omental adipose tissue *NNMT* expression with insulin sensitivity was found in subjects with insulin resistance (GIR<50 µmol/kg/min).
Fig. 4(C) shows the correlation of *NNMT* expression in omental adipose tissue and plasma 1-methylnicotinamide concentrations in subjects with GIR<50 µmol/kg/min. A positive correlation between *NNMT* expression in omental adipose tissue and plasma 1-methylnicotinamide concentrations was found in insulin-resistant subjects.
Fig. 4(D) shows the correlation of GIR and plasma MNA concentrations in subjects with GIR<50 µmol/kg/min. A negative correlation of GIR and plasma MNA was found in insulin-resistant subjects.
Figure 5: Changes in *NNMT* expression in adipose tissue upon exercise intervention.
Fig. 5(A) shows the changes in *NNMT* expression in subcutaneous adipose tissue before and after exercise intervention for three different groups of subjects: subjects with normal glucose tolerance (NGT), impaired glucose tolerance (IGT) or T2D (N=20 each; **p<0.001 vs pre-exercise, paired t-test).
Fig. 5(B) shows intra-individual changes in *NNMT* expression in subjects with normal glucose tolerance (NGT), impaired glucose tolerance (IGT) or T2D (percentage of pre-exercise expression, **p<0.001 vs. NGT, paired t-test).
Fig 5(C) shows the correlation between adipose tissue *NNMT* expression and glucose infusion rate during hyperinsulinemic-euglycemic clamp before the exercise program.
Fig. 5(D) shows the correlation between adipose tissue *NNMT* expression and glucose infusion rate during hyperinsulinemic-euglycemic clamp after the exercise program.
Figure 6: Correlation between MNA concentrations and *NNMT* expression in adipose tissue dependent on BMI.
Fig. 6(A) shows the correlation between plasma MNA concentrations and *NNMT* expression in omental adipose tissue for subjects with BMI below median (BMI<39, N=99).
Fig. 6(B) shows the correlation between plasma MNA concentrations and *NNMT* expression in subcutaneous adipose tissue for subjects with BMI<39.
Fig. 6(C) shows the correlation between plasma MNA concentrations and *NNMT* expression in omental adipose tissue for subjects with BMI>39 (N=99).
Fig. 6(D) shows the correlation between plasma MNA concentrations and *NNMT* expression in subcutaneous adipose tissue for subjects with BMI>39.
Figure 7 shows the correlation between GIR and subcutaneous adipose tissue *NNMT* expression in subjects with insulin resistance (GIR<50 µmol/kg/min). A negative correlation of subcutaneous adipose tissue *NNMT* expression with insulin sensitivity was found in these subjects.
Figure 8: Decrease in plasma 1-methylnicotinamide concentrations upon administration of an NNMT inhibitor

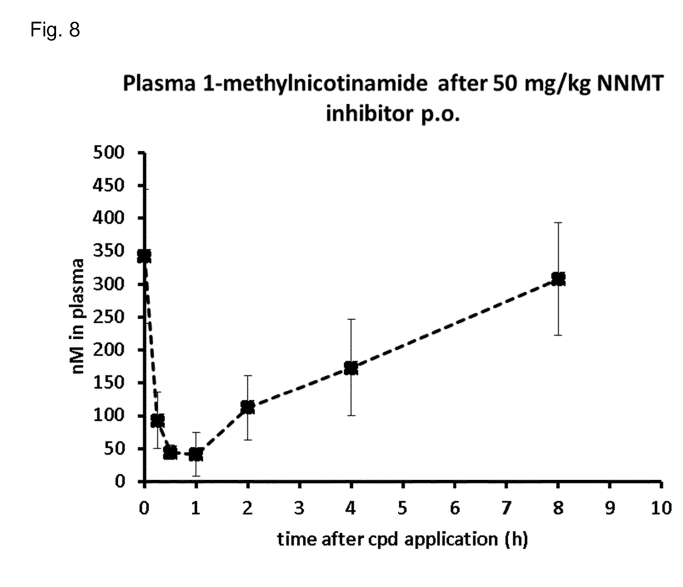

Fig. 8 shows the time-dependent concentration of 1-methylnicotinamide in plasma after administration of an NNMT inhibitor. A small molecule NNMT inhibitor was administered as a 50 mg/kg oral bolus to female C57B/L6 mice. After 0.25, 0.5, 1, 2, 4, 8, 16 and 24 hours, animals were killed (n=3 per time point) and 1-methylnicotinamide concentrations were determined in plasma as described. There was a rapid drop in plasma 1-MNA by about 90 % within 30 min, and concentrations returned to normal within eight hours.

### DETAILED DESCRIPTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art to which this invention belongs.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

Several documents (for example: patents, patent applications, scientific publications, manufacturer's specifications, instructions, GenBank Accession Number sequence submissions etc.) are cited throughout the text of this specification. Nothing herein is to be construed as an admission that the invention is not entitled to antedate such disclosure by virtue of prior invention. Some of the documents cited herein are characterized as being *"incorporated by reference".* In the event of a conflict between the definitions or teachings of such incorporated references and definitions or teachings recited in the present specification, the text of the present specification takes precedence.

The term "about" when used in connection with a numerical value is meant to encompass numerical values within a range having a lower limit that is 5% smaller than the indicated numerical value and having an upper limit that is 5% larger than the indicated numerical value.

The body mass index (abbreviated as BMI) is calculated by dividing the subject's weight (measured in kg) by his or her squared height (measured in m). Accordingly, the BMI is measured in kg/m². According to the World Health Organization (WHO), the normal range for the BMI is between 18.50 and 24.99. Subjects with a BMI in the range between 25.00 and 29.99 are regarded as overweight; subjects with a BMI in the range between 30.00 and 34.99 are grouped into obese class I; subjects with a BMI in the range between 35.00 and 39.99 are grouped into obese class II; and subjects with a BMI ≥ 40.00 are grouped into obese class III.

As used herein, the expressions "inhibitor of nicotinamide-N-methyltransferase" or "inhibitor of NNMT" or NNMT inhibitor" refer to a substance that inhibits (e.g. decreases or eliminates) the production or activity of NNMT mRNA or NNMT protein. NNMT activity includes, without limitation, catalysing the N-methylation of nicotinamide to produce 1-methylnicotinamide (MNA). In some embodiments, the "inhibitor of NNMT" inhibits the expression of the NNMT gene or the production of an NNMT gene product, thereby reducing the level of active NNMT protein in a cell. In some other embodiments, the "inhibitor of NNMT" inhibits an activity of the NNMT protein, for example by directly binding to the NNMT protein and by interfering with its function; e.g. as a competitive inhibitor or as a non-competitive inhibitor.

As used herein, the terms "level of 1-methylnicotinamide", "level of MNA" or "MNA level" refer to the concentration of 1-methylnicotinamide (MNA) in a bodily sample, especially in a whole blood sample, a serum sample or a plasma sample.

As used herein, the term "small molecule" encompasses organic compounds, organometallic compounds, inorganic compounds, and salts of organic, organometallic or inorganic compounds. "Small molecules" typically have a molecular weight of 5000 daltons or less, particularly 2000 daltons or less, and especially 1000 daltons or less. "Small molecules" may be found in nature (e.g. identified, isolated, purified) or may be produced synthetically (e.g. by traditional organic synthesis, bio-mediated synthesis, or a combination thereof), Examples of small molecules include, without limitation, hormones, neurotransmitters, nucleotides, amino acids, sugars, lipids, and their derivatives.

As used herein, the term "peptide" encompasses any molecule comprising at least one peptide bond. The peptide may contain L-amino acids and D-amino acids and any combination thereof. The amino acids present in the peptide may be common α-amino acids (e.g. glycine, alanine, valine, leucine, etc.), or non-α-amino acids (e.g. β-alanine, 4-aminobutyric acid, 6-aminocaproic acid, sarcosine, statine) or unusual amino acids (e.g. citrulline, homocitrulline, ornithine, homoserine, norleucine, norvaline) or any combination thereof. A peptide may be linear, branched or cyclic.

In some embodiments of the invention, the inhibitor of NNMT is a nucleic acid. "Nucleic acids" that are suitable inhibitors in the context of the present invention include, without limitation, antisense oligonucleotides (ASOs), small interfering RNAs (siRNA), short hairpin RNAs (shRNAs), microRNAs, ribozymes and aptamers. In some embodiments, a nucleic acid NNMT inhibitor inhibits (e.g. decreases or eliminates) the expression of an NNMT gene or an NNMT mRNA. In some embodiments, a nucleic acid NNMT inhibitor inhibits (e.g. decreases or eliminates) translation of an NNMT mRNA. Typically, a nucleic acid NNMT inhibitor achieves inhibition of transcription or translation by hybridizing to an NNMT gene or RNA.

As used herein, an antisense oligonucleotide (ASO) may comprise naturally occurring nucleotide subunits and/or nucleotide analogues and/or nucleotide mimetics. Thus, antisense oligonucleotides can comprise DNA, RNA, locked nucleic acid, PNA, glycol nucleic acid (GNA), threose nucleic acid (TNA), or morpholino nucleic acid analogs.

"Antibodies" and "antigen-binding fragments thereof" suitable for use in the present invention include, but are not limited to, polyclonal, monoclonal, monovalent, bispecific, heteroconjugate, multispecific, recombinant, heterologous, heterohybrid, chimeric, humanized (in particular CDR-grafted), deimmunized, or human antibodies, Fab fragments, Fab' fragments, F(ab')₂ fragments, fragments produced by a Fab expression library, Fd, Fv, disulfide-linked Fvs (dsFv), single chain antibodies (e.g. scFv), diabodies or tetrabodies, nanobodies (also known as single domain antibodies), anti-idiotypic (anti-Id) antibodies (including, e.g., anti-Id antibodies to antibodies of the invention), and epitope-binding fragments of any of the above.

In the context of the present invention, a "reference value" refers to a comparative value that represents the concentration of a biological marker in a healthy subject. This reference value may be a defined numerical value that has usually been determined empirically. Such a defined numerical value is also often termed "threshold value". Alternatively, a "reference value" may be determined by obtaining samples from one or more (e.g. 2, 3, 4, 5, 6, 7, 8, 9, or 10) healthy subjects, by measuring the concentration of the desired biological marker in these samples and by calculating the average concentration of the biological marker in the samples from the healthy subjects.

In the context of the present invention, a "pre-treatment value" refers to a comparative value that represents the concentration of a biological marker in a patient prior to treatment with a medicament.

The abbreviation T2D means "type 2 diabetes mellitus".

The abbreviation IGT means "impaired glucose tolerance". According to the criteria of the World Health Organization and the American Diabetes Association, impaired glucose tolerance is defined as: two-hour glucose levels of 140 to 199 mg per dL (7.8 to 11.0 mmol) on the 75-g oral glucose tolerance test. A patient is said to be under the condition of IGT when he/she has an intermediately raised glucose level after 2 hours, but less than would qualify for type 2 diabetes mellitus. The fasting glucose may be either normal or mildly elevated.

The abbreviation NGT means "normal glucose tolerance". Normal glucose tolerance is defined as: two-hour glucose levels of less than 140 mg per dL (less than 7.8 mmol) on the 75-g oral glucose tolerance test. Fasting plasma glucose (measured before the oral glucose tolerance test begins) should be below 110 mg/dL (below 6.1 mmol/L).

The abbreviation WAT means "white adipose tissue", also known as white fat.

As used herein, the expression "visceral WAT" refers to white adipose tissue that is located inside the peritoneal cavity, packed in between internal organs and torso, as opposed to subcutaneous fat, which is found underneath the skin, and intramuscular fat, which is found interspersed in skeletal muscle.

A more specific type of "visceral WAT" is "omental WAT". "Omental WAT" is white adipose tissue that is located in the greater omentum or in the lesser omentum. The greater omentum (also the great omentum, omentum majus, gastrocolic omentum, epiploon, or, especially in animals, caul) is a large apron-like fold of visceral peritoneum that hangs down from the stomach. It extends from the greater curvature of the stomach, passing in front of the small intestines and reflects on itself to ascend to the transverse colon before reaching to the posterior abdominal wall. The lesser omentum (small omentum; gastrohepatic omentum; Latin: omentum minus) is the double layer of peritoneum that extends from the liver to the lesser curvature of the stomach and the start of the duodenum.

As used herein, the expression *"a lack of decrease of the level of MNA in the blood sample when compared to the pre-treatment level of MNA*" encompasses the following two alternatives: (i) an increase of the level of MNA in the blood sample when compared to the reference level of the MNA or (ii) an identical level of MNA in the blood sample when compared to the reference level of the MNA.

As used herein, a "patient" means any mammal or bird who may benefit from a treatment with an inhibitor of NNMT described herein. Preferably, a "patient" is selected from the group consisting of laboratory animals (e.g. mouse or rat), domestic animals (including e.g. guinea pig, rabbit, chicken, turkey, pig, sheep, goat, camel, cow, horse, donkey, cat, or dog), or non-human primates (e.g. African green monkeys, chimpanzees, bonobos, gorillas) and human beings. It is particularly preferred that the "patient" is a human being.

As used herein, the term "healthy subject" refers to a subject with normal glucose tolerance. In other words, a "healthy subject" refers to a subject who does not have type 2 diabetes and who does not have an impaired glucose tolerance. In the context of the present invention, the usage of the term "healthy subject" does not preclude the possibility that the subject suffers from other diseases that are unrelated to diabetes or to glucose metabolism.

A "therapeutically effective amount" is an amount of a therapeutic agent sufficient to achieve the intended purpose. The therapeutically effective amount of a given therapeutic agent will vary with factors such as the nature of the agent, the route of administration, the size and species of the subject to receive the therapeutic agent, and the purpose of the administration. The therapeutically effective amount in each individual case may be determined empirically by a skilled artisan according to established methods in the art.

"Pharmaceutically acceptable" means approved by a regulatory agency of the Federal or a state government or listed in the U.S. Pharmacopeia (United States Pharmacopeia-37/National Formulary-32, published by the United States Pharmacopeial Convention, Inc., Rockville Md., publication date: 1 November 2013) or other generally recognized pharmacopeia for use in animals, and more particularly in humans.

### Embodiments of the Invention

The present invention will now be further described. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous, unless clearly indicated to the contrary.

In a first aspect the present invention is directed to a method of determining whether a patient with insulin resistance or type-2 diabetes (T2D) will benefit from a therapeutic treatment with an inhibitor of nicotinamide-N-methyltransferase (NNMT), said method comprising the steps:
- determining the level of 1-methylnicotinamide (MNA) in a blood sample isolated from the patient; and
- comparing the level of MNA in the blood sample with a reference level of MNA;
wherein an increased MNA level in the blood sample isolated from the patient as compared to the reference level of MNA indicates that the patient will benefit from said therapeutic treatment.

In one embodiment of the first aspect, the reference level is an average MNA level determined from at least two (e.g. 2, 3, 4, 5, 6, 7, 8, 9, 10, 11, 12, 13, 14, 15, 16, 17, 18, 19, 20, 25, 30, 35, 40, 45, 50) reference samples from healthy subjects (healthy controls). In a further embodiment of the first aspect, an increase of the MNA level in the blood sample isolated from the patient by at least 20% (e.g. at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, or at least 120%) as compared to the reference level of MNA indicates that the patient will benefit from said therapeutic treatment.

In another embodiment of the first aspect, the reference level is at least 100 nmol/L (e.g. at least 150 nmol/L, at least 200 nmol/L, at least 250 nmol/L, at least 300 nmol/L, at least 350 nmol/L, at least 400 nmol/L).

In one embodiment of the first aspect, the method comprises the further step of administering a therapeutically effective amount of an inhibitor of NNMT to a patient for whom it has been determined that the patient will benefit from said therapeutic treatment.

In a second aspect the present invention is directed to a method of determining whether a patient with insulin resistance or type-2 diabetes (T2D) responds to a therapeutic treatment with an inhibitor of nicotinamide-N-methyltransferase (NNMT), said method comprising the steps:
- determining the level of 1-methylnicotinamide (MNA) in a blood sample isolated from the patient; and
- comparing the level of MNA in the blood sample with a pre-treatment level of MNA; wherein a decrease of the MNA level in the blood sample isolated from the patient as compared to the pre-treatment level of MNA indicates that the patient responds to said therapeutic treatment; and
wherein the patient is a patient to whom at least once an inhibitor of NNMT is administered or has been administered.

In one embodiment of the second aspect, the blood sample is isolated from the patient after an inhibitor of NNMT has been administered to said patient.

In one embodiment of the second aspect, the pre-treatment level is determined in a blood sample isolated from the patient before an inhibitor of NNMT has been administered to said patient. In a further embodiment of the second aspect, a decrease of the MNA level by at least 10% (e.g. at least 15%, at least 20%, at least 25%, at least 30%, at least 35%, at least 40%, at least 45%, at least 50%, at least 55%, at least 60%, at least 65%, at least 70%, at least 75%, at least 80%) in the blood sample isolated from the patient as compared to the pre-treatment level of MNA indicates that the patient responds to said therapeutic treatment.

In one embodiment of the second aspect, the method comprises the further step of administering (again) a therapeutically effective amount of an inhibitor of NNMT to a patient for whom it has been determined that the patient responds to said therapeutic treatment.

In a third aspect the present invention is directed to a method of adjusting the dose of a therapeutic drug applied for therapeutic treatment of insulin resistance or type-2 diabetes (T2D) in a patient comprising the steps:
- determining the level of 1-methylnicotinamide (MNA) in a blood sample isolated from the patient; and
- comparing the level of MNA in the blood sample with a pre-treatment level of MNA; wherein the patient is a patient to whom at least once an inhibitor of NNMT is administered or has been administered; and
wherein said therapeutic drug is an inhibitor of nicotinamide-N-methyltransferase (NNMT).

In one embodiment of the third aspect, the blood sample is isolated from the patient after an inhibitor of NNMT has been administered to said patient.

In one embodiment of the third aspect, the pre-treatment level is determined in a blood sample isolated from the patient before an inhibitor of NNMT has been administered to said patient.

In a further embodiment of the third aspect, a decrease of the level of MNA in the blood sample when compared to the pre-treatment level of MNA indicates that the dose of the therapeutic drug applied for treating T2D in the patient is sufficient and can optionally be (further) lowered. In further embodiments, the method comprises the further step of administering a therapeutic drug (e.g. an inhibitor of NNMT) to the patient in a dose that is lower than the last dose that had been administered to the patient.

In a further embodiment of the third aspect, a lack of decrease of the level of MNA in the blood sample when compared to the pre-treatment level of MNA indicates that the dose of the therapeutic drug applied for treating T2D in the patient is not sufficient and can optionally be increased (again). In further embodiments, the method comprises the further step of administering a therapeutic drug (e.g. an inhibitor of NNMT) to the patient in a dose that is higher than the last dose that had been administered to the patient.

In some embodiments of the third aspect, the level of MNA in the blood sample can be decreased when compared to the pre-treatment level of MNA but this decrease may be insufficient to achieve the intended therapeutic effect. For example, the patient's MNA level may be decreased but the patient's insulin sensitivity may not have substantially increased as compared to the situation before begin of the treatment. In these embodiments, the dose of the therapeutic drug applied for treating T2D in the patient is not sufficient to achieve the intended therapeutic effect and can optionally be increased. In further embodiments, the method comprises the further step of administering a therapeutic drug (e.g. an inhibitor of NNMT) to the patient in a dose that is higher than the last dose that had been administered to the patient.

In one embodiment of the first, second or third aspect, the inhibitor of NNMT is selected from the group consisting of small molecules, peptides, nucleic acids, and antibodies.

In some embodiments of any aspect of the present invention, the nucleic acid is selected from the group consisting of an antisense oligonucleotide (ASO), a small interfering RNA (siRNA), a short hairpin RNA (shRNA), a microRNA, an aptamer and a ribozyme.

In a fourth aspect the present invention is directed to a method for determining the degree of insulin resistance in a patient, comprising the steps:
- determining the level of MNA in a blood sample isolated from the patient; and
- comparing the level of MNA in the blood sample with a reference level of MNA;
wherein the degree of the increase of the MNA level in the blood sample isolated from the patient as compared to the reference level of MNA is indicative for the degree of insulin resistance in the patient.

In one embodiment of the fourth aspect, the reference level is an average MNA level determined from at least two (e.g. 2, 3, 4, 5, 6, 7, 8, 9, 10, 11, 12, 13, 14, 15, 16, 17, 18, 19, 20, 25, 30, 35, 40, 45, 50) reference samples from healthy subjects (healthy controls).

In another embodiment of the fourth aspect, the reference level is at least 100 nmol/L (e.g. at least 150 nmol/L, at least 200 nmol/L, at least 250 nmol/L, at least 300 nmol/L, at least 350 nmol/L, at least 400 nmol/L).

In one embodiment of the fourth aspect, the degree of insulin resistance in the patient determined by the method provides the basis for a further therapeutic decision. For example, depending on the degree of insulin resistance, a selection of drugs to be administered (e.g. insulin; insulin sensitizer etc.) may be made and/or the dosage of a drug may be adjusted.

In one embodiment of the first, second, third or fourth aspect, the patient is a mammal, preferably a human, a non-human primate or a rodent.

In one embodiment of the first, second, third or fourth aspect, the patient is a human and has a body mass index (BMI) of 25 kg/m² or more; for example 30 kg/m² or more, 35 kg/m² or more, or 40 kg/m² or more.

In one embodiment of the first, second, third or fourth aspect, the level of MNA in the blood sample is determined by mass spectrometry. In some embodiments, the mass spectrometer is an electrospray ionization mass spectrometer (ESI-MS), e.g. an electrospray ionization-triple quadrupole mass spectrometer. In one embodiment, the mass spectrometer comprises an H-electrospray ionization (HESI) source, which may be operated in the positive ion mode. In further embodiments, the mass spectrometer is coupled to a liquid chromatography system. In some embodiments, the chromatographic separation is achieved with two eluents: a first eluent (A) comprising or consisting of 0.1 % formic acid in water; and a second eluent (B) comprising or consisting of 0.1% formic acid in acetonitrile. In further embodiments, separation is achieved by an initial chromatography step of 98%B (and correspondingly 2%A), followed by a gradient to 5%B (and correspondingly 95%A), and followed by a further step of 5% B (and correspondingly 95% A). In some embodiments, quantification is performed via peak area ratios (SRM m/z 137 -> m/z 94) applied to internal standard d4-N-methylnicotinamide (SRM m/z 141 -> m/z 98) in an external calibration curve.

In one embodiment of the first, second, third or fourth aspect, the blood sample is a plasma sample or a serum sample.

### EXAMPLES

The following examples are put forth so as to provide those of ordinary skill in the art with a complete disclosure and description of how to make and use the methods and uses of the invention, and are not intended to limit the scope of what the inventors regard as their invention. Efforts have been made to ensure accuracy with respect to numbers used but some experimental errors and deviations should be accounted for. Unless indicated otherwise, molecular weight is average molecular weight, temperature is in degrees Celsius, and pressure is at or near atmospheric.

### Subjects and Methods

### Study population

We included three different cohorts with a total number of 273 individuals in our study of MNA serum concentration and *NNMT* adipose tissue mRNA expression. In the first cross-sectional cohort (n=199; 128 women, 71 men), we investigated MNA serum concentrations and *NNMT* mRNA expression in paired samples of subcutaneous and omental adipose tissue in relation to measures of obesity and glucose metabolism. Individuals involved in these analyses underwent abdominal surgery for weight reduction surgery, cholecystectomy, abdominal injuries or explorative laparotomy between 2009 and 2012. All subjects had a stable weight, defined as the absence of fluctuations of >2% of body weight for at least 3 months before surgery. Adipose tissue was immediately frozen in liquid nitrogen after explantation. Histologic analyses and measurement of macrophage count in adipose tissue was performed as previously described (Harman-Boehm et al, J Clin Endocrinol Metab. 2007 92:2240-7, the content of which is herein incorporated by reference in its entirety).

In a second interventional study, we investigated circulating MNA in response to a 12 weeks intensive exercise intervention in 60 individuals with different degrees of glucose tolerance. Individuals of all cohorts fulfilled the following inclusion criteria: 1) Absence of any acute or chronic inflammatory disease as determined by a leucocyte count > 8000 Gpt/l, C-reactive protein (CrP) > 10.0 mg/dl or clinical signs of infection, 2) Undetectable antibodies against glutamic acid decarboxylase (GAD), 3) Systolic blood pressure (SBP) < 140mmHg and diastolic blood pressure (DBP) < 90mmHg, 4) No clinical evidence of either cardiovascular or peripheral artery disease, 5) No thyroid dysfunction, 6) No alcohol or drug abuse, 7) No pregnancy.

All study protocols have been approved by the ethics committee of the University of Leipzig (Reg.No. 031-2006 and 017-12-23012012). All participants gave written informed consent before taking part in the study.

### Exercise intervention

Sixty subjects were divided into groups of normal glucose tolerance (NGT, n=20; 9 males, 11 females), impaired glucose tolerance (IGT, n=20; 9 males, 11 females), and type 2 diabetes (T2D, n=20; 11 males, 9 females) on the basis of a 75g oral glucose tolerance test (OGTT) according to the ADA-Criteria. Subjects with NGT were defined by a fasting plasma glucose <6.0mmol/l, and a 120min plasma glucose <7.8mmol/l. Subjects with IGT were defined by a fasting plasma glucose <6.0mmol/l, a 120min plasma glucose > 7.8mmol/l and <11.1mmol/l. Subjects with T2D were defined by a fasting plasma glucose > 7.0mmol/l and/or a 120min OGTT glucose >11.1 mmol/l. These 60 individuals were enrolled in 60 minutes of supervised physical training sessions 3 days per week. In brief, each training session included 20 minutes of biking or running, 20 minutes of swimming, and 20 minutes of warming up/cooling down periods. All subjects completed a graded bicycle-ergometer test to volitional exhaustion and had maximal oxygen uptake measured with an automated open circuit gas analysis system at baseline, after 4 and 12 weeks of training. The highest oxygen uptake/minute reached was defined as the maximal oxygen uptake, and subjects subsequently trained at their individual submaximal heart rate defined as 70-80% of the individual maximal heart rate during the bicycle-ergometer test. At baseline, after 4 and 12 weeks of training (48 hours after the last training session), blood samples were obtained in the fasting state and measurements of anthropometric parameters were performed. Subcutaneous adipose tissue biopsies were taken before and after the exercise program.

### Measurement of body fat content, glucose metabolism, insulin sensitivity

BMI was calculated as weight divided by squared height. Hip circumference was measured over the buttocks; waist circumference was measured at the midpoint between the lower ribs and iliac crest. Percentage body fat was measured by dual X-ray absorptiometry (DEXA). Abdominal visceral and subcutaneous fat areas were calculated using magnet resonance images (MRI) or computed tomography (CT) scans at the level of L4-L5 in the cross-sectional study of adipose tissue donors. Three days before the OGTT, patients documented a high-carbohydrate diet in diet protocols. The OGTT was performed after an overnight fast with 75 g standardized glucose solution (Glucodex Solution 75 g; Merieux, Montreal, Canada). Venous blood samples were taken at 0, 60, and 120 min for measurements of plasma glucose concentrations. Insulin sensitivity was assessed using the HOMA-IR index or with the euglycemic-hyperinsulinemic clamp method as described (Blüher et al. Diabetologia 2002 45:210-6, the content of which is herein incorporated by reference in its entirety).

### Analyses of blood samples

All baseline blood samples were collected between 8 and 10 am after an overnight fast. Plasma insulin was measured with an enzyme immunometric assay for the IMMULITE automated analyzer (Diagnostic Products Corporation, Los Angeles, CA, USA). Serum lipid profiles were measured as previously described (Klöting et al. Am J Physiol Endocrinol Metab. 2010 299:E506-15, the content of which is herein incorporated by reference in its entirety).

### NNMT mRNA expression studies

Human *NNMT* mRNA expression was measured by quantitative real-time RT-PCR in a fluorescent temperature cycler using the TaqMan assay, and fluorescence was detected on an ABI PRISM 7000 sequence detector (Applied Biosystems, Darmstadt, Germany). Samples of subcutaneous and omental adipose tissue were immediately frozen in liquid nitrogen after biopsy. Total RNA was isolated using TRlzol® (Life Technologies, Grand Island, NY), and 1 µg RNA was reverse transcribed with standard reagents (Life Technologies). From each RT-PCR, 1 µl was amplified in a 20-µl PCR using the Brilliant® SYBR® Green QPCR Core Reagent kit from Stratagene (La Jolla, CA) according to the manufacturer's instructions. *NNMT* mRNA expression was measured by quantitative real-time RT-PCR in a fluorescent temperature cycler using a TaqMan® Gene Expression Assay (Hs00196287_m1) that contains premixed fluorescently labelled probes and TaqMan® 2xUniversal PCR Master Mix (Applied Biosystems, Darmstadt, Germany). Samples were incubated in the ABI PRISM 7000 sequence detector for an initial denaturation at 95°C for 10 min, followed by 40 PCR cycles, each cycle consisting of 95°C for 15 s, 60°C for 1 min, and 72°C for 1 min. Fluorescence emissions were monitored after each cycle. *NNMT* mRNA expression was calculated relative to the mRNA expression of Hypoxanthine-Guanine-Phosphoribosyltransferase 1 (HPRT1), determined by a pre-developed TaqMan® Assay (Hs01003267_m1; Applied Biosystems): Normalization by endogenous control results in arbitrary units (AU).

### LCMSMS measurements for quantification of MNA concentrations in plasma

Analysis was performed using an electrospray ionization-triple quadrupol mass spectrometer (Quantum Ultra,Thermo) coupled to a liquid chromatography system (UltiMate3000, Dionex) controlled by XCalibur 2.0.7 software with Dionex Chrom MS link 6.80. Chromatographic separation was achieved on a Kinetex Hilic 2.6µM 2,1x100mm analytical column (Phenomenex) at 65°C with a flow rate of 350µL/min; autosampler temperature was set to 10°C. A sample volume of 20µL was injected onto the column. Eluents consisted of water/0.1% formic acid (A) and acetonitrile/0.1%formic acid (B). Initial conditions (0-0.85min) were 98% B, then a ramp was applied within 1.1 min to 5%B, a conditions which remained until 3.25 min, before the system was equilibrated to initial conditions within 2.15 min, resulting in a total run time of 5.5min per sample. The column flow was directly converted into the H-electrospray ionization (HESI) source of the mass spectrometer, which was operated in the positive ion mode. Capillary and vaporizer temperatures were maintained at 320°C and 350°C, respectively. Sheath gas and auxiliary gas were operated at 60 and 15 (pressure, arbitrary units), no ion sweep gas was applied. Collision energy, single reaction monitoring and tube lens offset were adjusted to 19 V and 70 V, respectively. Quantification was performed via peak area ratios (SRM m/z 137 -> m/z 94) applied to internal standard d4-N-methylnicotinamide (SRM m/z 141 -> m/z 98) in an external calibration curve using XCalibur QuanBrowser.

Sample preparation started with protein precipitation by addition of 80µL of a freshly prepared acetonitrile solution containing 0.25 µM d4-NMA to 20µL plasma sample in a 0.5mL plastic tube. After rigorous mixing the samples were incubated at 5°C in the refrigerator for 20 min before they were centrifuged at 16100 rcf at 4°C for 20min. 60µL of the supernatant was transferred to a fresh vial which was then tightly sealed before undergoing analysis. 60 samples per day were prepared and analyzed overnight. Each day, samples for a daily external calibration curve were prepared by using the exact acetonitrile/0.25µM d4-MNA- lot which has been used for the samples. Thus, the eight calibration samples contained 0.25µM internal standard different amounts of MNA ranging from 0 - 1000nM.

### Statistical analysis

The homoscedastic two-sided Student's t-test was used to compare clinical characteristics between different groups of subjects, e.g., *NNMT* expression between non-diabetic and diabetic subjects. The paired t-test was used to compare clinical parameters within individual subjects, e.g. adipose tissue NNMT expression before and after exercise intervention or before and after bariatric surgery. *P* values < 0.05 were considered to be statistically significant.

Spearman's correlation coefficients were calculated to evaluate the relationships between different clinical characteristics among the same group of individuals, e.g., to investigate the association between plasma MNA concentrations and adipose tissue *NNMT* expression. For the latter, data were log-transformed to achieve normal distribution.

### Change in plasma 1-methylnicotinamide concentrations upon administration of an NNMT inhibitor

A small molecule NNMT inhibitor was administered as a 50 mg/kg oral bolus to female C57B/L6 mice. After 0.25, 0.5, 1, 2, 4, 8, 16 and 24 hours, animals were killed (n=3 per time point) and 1-methylnicotinamide concentrations were determined in plasma as described above.

### Results

Anthropometric, demographic and clinical characteristics of the study participants are summarized in Table 1. Overall, adipose tissue and plasma samples were collected from 199 subjects undergoing abdominal surgery, 88 of them having been diagnosed with T2D before surgery. The mean age of the study participants at the time of surgery was 55 ± 16 years, with no statistically significant difference between non-diabetic and diabetic subjects. In both sub-populations, about two thirds of the study subjects were women. Compared to the patients with T2D, non-diabetic subjects had significantly lower body weights, BMI and plasma triglyceride concentrations, and slightly higher HDL-C and LDL-C values. In line with the T2D diagnosis, fasting plasma glucose and HbA1c were significantly lower whereas fasting plasma insulin and glucose infusion rates in hyperinsulinemic-euglycemic clamp were significantly higher in the group without T2D.

**Table 1. Baseline anthropometric and clinical characteristics^{a} of study subjects before abdominal surgery (study 1)**

| | No T2D (n=111) | T2D(n=88) | P value^{b} |
|---|---|---|---|
| Age, y | 56 ± 18 | 53 ± 13 | .13 |
| Female, n (%) | 73 (66) | 55 (63) | .63 |
| Weight, kg | 95 ± 41 | 144 ± 41 | <.001 |
| BMI, kg/m² | 33 ± 13 | 49 ± 13 | <.001 |
| FPG^{b}, mM | 5.5 ± 0.9 | 7.3 ± 2.5 | <.001 |
| FPI^{d}, pM | 79 ± 108 | 247 ± 198 | .001 |
| HbA1c^{e}, % | 5.4 ± 0.4 | 6.6 ± 1.1 | <.001 |
| GIR^{f}, µmol/kg/min | 87 ± 28 | 35 ± 24 | <.001 |
| TG^{g}, mM | 1.2 ± 0.5 | 2.0 ± 1.0 | <.001 |
| LDL-C^{h}, mM | 3.1 ± 1.0 | 2.7 ± 0.8 | .04 |
| HDL-C^{h}, mM | 1.4 ± 0.5 | 1.2 ± 0.4 | .01 |

| | | | |
|---|---|---|---|
| ^{a} Data are given as mean ± standard deviation for parametric variables or number (column percentage) for categorical variables ^{b} P values were determined using Student's t-test for continuous parametric values and X² tests for categorical values ^{c} Fasting plasma glucose, n=175 (95 no T2D, 80 T2D) ^{d} Fasting plasma insulin, n=120 (58 no T2D, 62 T2D) ^{e} HbA1c%, n=110 (53 no T2D, 57 T2D) ^{f}Glucose infusion rate, n=85 (52 no T2D, 33 T2D) ^{g} Triglycerides, n=119 (52 no T2D, 67 T2D) ^{h}LDL-C, HDL-C, n=116 (51 T2D, 65 T2D) | | | |

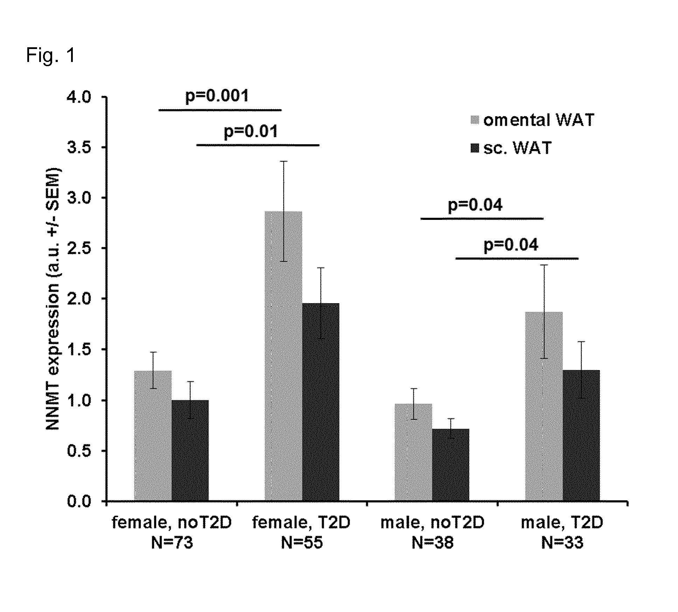

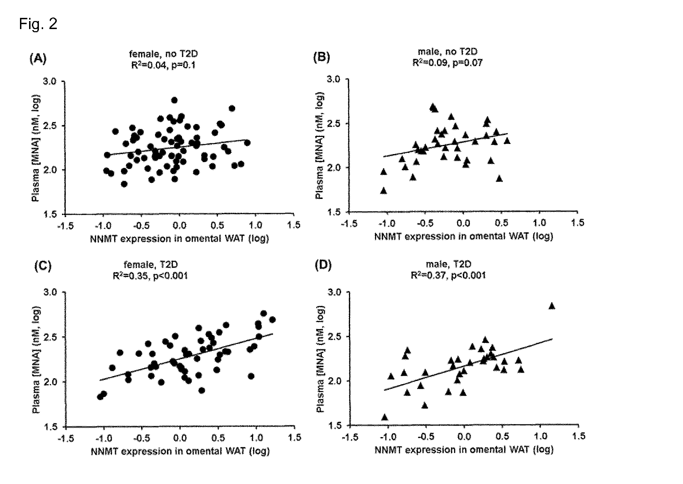

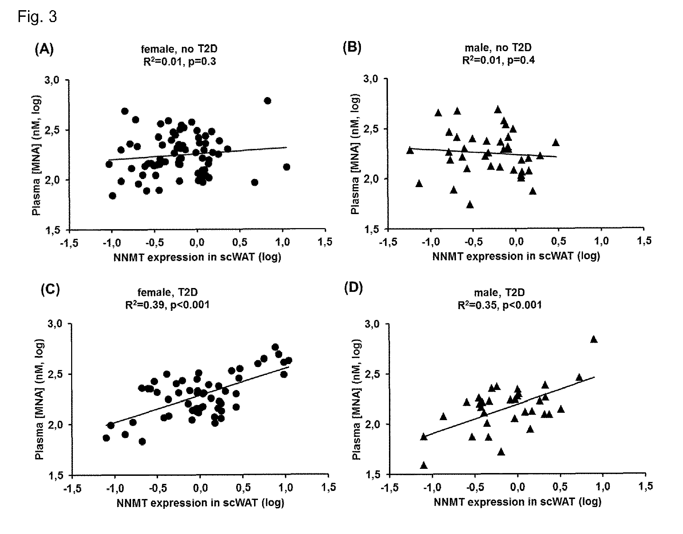

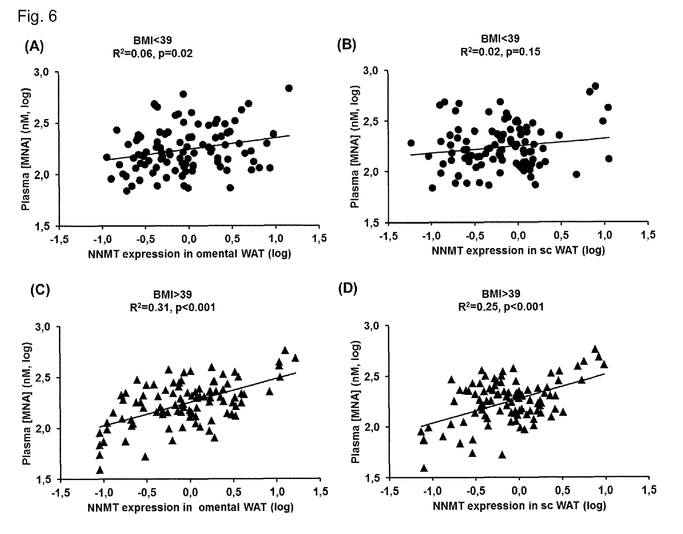

Figure 1 shows the association of NNMT expression in adipose tissue with the presence of T2D. In both subcutaneous and omental white adipose tissue, there was a significant, approximately two-fold increase in NNMT expression in the T2D group compared to the non-diabetic subjects, irrespective of gender. Assuming a log-normal distribution of NNMT expression, geometric mean ratios (95 % confidence interval) for T2D patients compared to non-diabetic subjects were determined as 1.82 (1.22-2.72) and 1.54 (0.91-2.60) for omental adipose tissue in females and males, respectively, and 1.67 (1.16- 2.41) and 1.52 (0.95-2.44) for subcutaneous adipose tissue in females and males, respectively. Interestingly, plasma MNA concentrations did not correlate with either omental (Figure 2A, 2B) or subcutaneous (Figure 3A, 3B) adipose tissue NNMT expression in non-diabetic subjects. However, there was a statistically significant positive association (R=0.6, p<0.001) between plasma MNA concentration and adipose tissue NNMT expression in both adipose depots and for both women and men (Figure 2C, 2D, 3C, 3D). The reason for this difference between non-diabetic and T2D patients is not clear. Potentially, in non-diabetic subjects a major part of MNA in plasma may come from the liver, with a lower contribution by adipose tissue, whereas in individuals with T2D adipose tissue may become a more predominant source of MNA. This would be in line with observations in mice on a high-fat diet where a selective increase in adipose tissue but not in liver NNMT activity was observed (Riederer et al, Atherosclerosis, 2009, 204:412-417). Interestingly, although there was no significant dependence of adipose tissue NNMT expression on BMI, MNA concentrations strongly correlated with omental or subcutaneous adipose tissue NNMT expression for subjects with a BMI above the median BMI of 39 (R=0.56 for omental, R=0.5 for subcutaneous adipose tissue NNMT, p<0.001 each) but less so for subjects with a BMI below median (R=0.24, p=0.02 for omental, R=0.15, p=0.15 for subcutaneous adipose tissue) which also points towards a higher contribution to MNA plasma levels of adipose tissue NNMT compared to liver NNMT in obese subjects (Figure 6C, 6D, 6A, 6B).

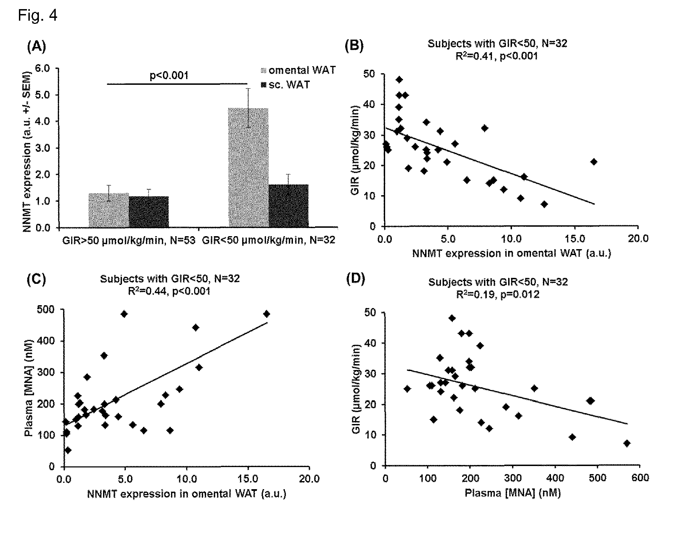

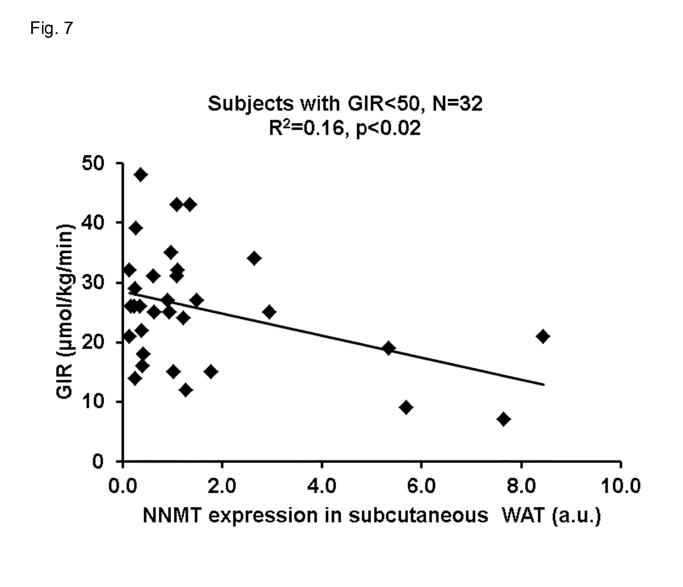

In subjects for which hyperinsulinemic-euglycemic clamp data were available (N=85), *NNMT* expression in omental adipose tissue was about 3.5-fold higher in subjects with insulin resistance (N=32, p<0.001) compared to insulin-sensitive individuals (N=53) where insulin resistance was defined by a glucose infusion rate (GIR) lower than 50 µmol/kg/min (Figure 4A). In contrast, the difference in subcutaneous adipose tissue *NNMT* mRNA between insulin-resistant and insulin-sensitive subjects did not reach statistical significance (p=0.3). In the group of insulin-resistant subjects, we observed a strong correlation between the extent of insulin resistance - defined by the lower GIR compared to insulin-sensitive subjects - and the expression level of *NNMT* in omental adipose tissue (Figure 4B, R=0.64, p<0.001). A similar but somewhat less pronounced (R=0.4, p=0.02) association was observed between low GIR and expression of *NNMT* in subcutaneous adipose tissue (Figure 7). As there was also a positive association between plasma MNA concentrations and adipose tissue *NNMT* expression in this study group (Figure 4C, R=0.66, p<0.001), plasma MNA levels significantly correlate with the degree of insulin resistance (Figure 4D, R=0.44, p=0.01). Thus, in insulin-resistant individuals, plasma MNA concentration could potentially be used as a soluble biomarker for adipose tissue *NNMT* expression and the extent of whole-body insulin sensitivity.

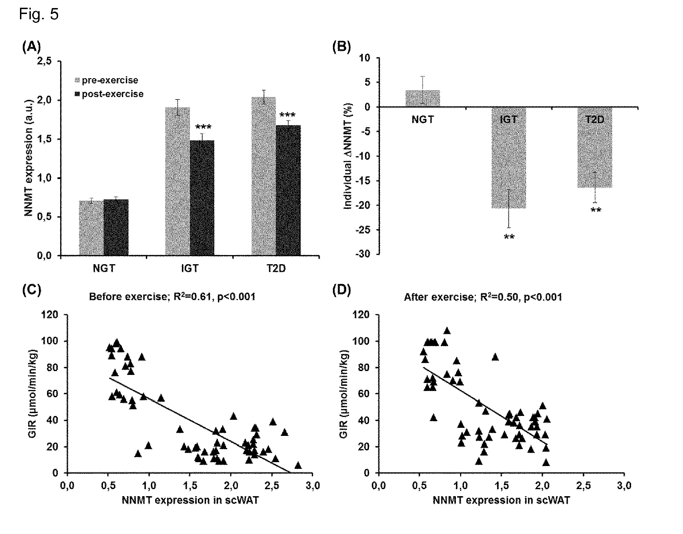

Of note, the association between adipose tissue NNMT expression and insulin resistance could be confirmed in a separate cohort (N=60, 20 each of NGT, IGT and T2D subjects) of subjects that enrolled for a 12-week exercise intervention program consisting of three one-hour bouts of exercise per week (20 min endurance and 40 min resistance training). Clinical and anthropometric characteristics of study subjects before and after exercise are summarized in Table 2. In all three groups - NGT, IGT and T2D - the physical training program led to a significant reduction in body weight, BMI, body fat content, plasma glucose levels two hours after oral glucose challenge, and basal insulin levels. As expected, compared to baseline, fasting plasma glucose concentrations were significantly lower after the exercise intervention in the T2D group (5.8 ± 0.5 vs. 6.2 ± 0.6 mM, p=0.007 in paired t-test) but not in the IGT and NGT groups. Furthermore, whole-body glucose uptake was significantly higher (p<0.001) in all three groups, with larger relative improvements observed in the IGT (36 ± 16 vs. 19 ± 9 µmol/kg/min) and T2D groups (32 ± 11 vs. 21 ± 9 µmol/kg/min) compared to the NGT group (85 ± 15 vs. 76 ± 17 µmol/kg/min). Of note, adipose tissue NNMT expression was again significantly higher in patients with T2D (2.9-fold, p<0.001) or IGT (2.7-fold, p<0.001) compared to people with NGT (Figure 5A). Importantly, the exercise program led to a significant decrease in adipose tissue NNMT expression in both IGT (-21 %, p<0.001) and T2D subjects (-16 %, p<0.001) but not in NGT individuals (+3 %, p=0.4, Figure 5B). There was a strong correlation between GIR and adipose tissue NNMT expression both before (Figure 5C, R=0.78, p<0.001) and after the exercise program (Figure 5D, R=0.70, p<0.001) confirming the association between tissue NNMT expression and the degree of insulin sensitivity that was observed in the larger cohort.

**Table 2. Anthropometric and clinical characteristics of exercise intervention cohort at baseline and after the 12-week exercise program^{a}**

| | NGT (N=20) | IGT (N=20) | T2D (N=20) |
|---|---|---|---|
| Age, y | 33 ± 11 | 56 ± 12*** | 53 ± 7*** |
| Female, n (%) | 11 (55) | 11 (55) | 9 (45) |
| Weight, kg, before/after exercise | 69.6 ± 14.4 | 87.6 ± 16.4*** | 94.7 ± 19.7*** |
| | 68.2 ± 13.7°° | 84.5 ± 16.2°°° | 93.0 ± 18.2°°° |
| BMI, kg/m², before/after exercise | 23.3 ± 3.2 | 29.6 ± 6.0*** | 32.2 ± 6.0*** |
| | 22.8 ± 3.1°° | 28.5±5.9°°° | 31.7 ± 5.5° |
| Body fat, %, before/after exercise | 24.5 ± 3.2 | 34.9 ± 8.3*** | 38.2 ± 8.0*** |
| | 23.3 ± 2.7°°° | 31.5 ± 7.5°°° | 35.2 ± 7.7°°° |
| FPG, mM, before/after exercise | 5.2 ± 0.5 | 5.7 ± 0.6** | 6.2 ± 0.6***^{,§§} |
| | 5.0 ± 0.4 | 5.5 ± 0.6 | 5.8 ± 0.5°° |
| Two-hour glucose, mM, before/after exercise | 6.0 ± 0.8 | 9.4 ± 0.9*** | 13.1 ± 1.5***^{,§§§} |
| | 5.6 ± 0.6°° | 8.1 ± 1.4°°° | 12.6 ± 2.5 |
| Basal insulin, pM, before/after exercise | 66 ± 35 | 695 ± 493*** | 319 ± 212***^{,§§} |
| | 58 ± 27° | 379 ± 324°°° | 234 ± 120°° |
| Whole-body glucose uptake, µmol/kg/min, before/after exercise | 76 ± 17 | 19 ±9*** | 21 ±9*** |
| | 85 ± 15°°° | 36 ± 16°°° | 32 ±11°°° |

| | | | |
|---|---|---|---|
| ^{a} Data are given as mean ± standard deviation for parametric variables or number (column percentage) for categorical variables ** p<0.01, *** p<0.001 vs. NGT (t-test) ^{§§} p<0.01, ^{§§§} p<0.001 vs. IGT (t-test) ° p>0.05, °° p<0.01, °°° p<0.001 vs. before exercise (paired t-test) | | | |

Fig. 8 shows the time-dependent concentration of 1-methylnicotinamide (1-MNA) in plasma after administration of an NNMT inhibitor to C57B/L6 mice. There was a rapid drop in plasma 1-MNA by about 90 % within 30 min, and concentrations returned to normal within eight hours.

## Claims

1. A method of determining whether a patient with insulin resistance or type-2 diabetes (T2D) will benefit from a therapeutic treatment with an inhibitor of nicotinamide-N-methyltransferase (NNMT), said method comprising the steps:
- determining the level of 1-methylnicotinamide (MNA) in a blood sample isolated from the patient; and
- comparing the level of MNA in the blood sample with a reference level of MNA;
wherein an increased MNA level in the blood sample isolated from the patient as compared to the reference level of MNA indicates that the patient will benefit from said therapeutic treatment.

2. The method of claim 1, wherein the reference level is an average MNA level determined from at least two reference samples from healthy subjects.

3. The method of claim 1 or 2, wherein an increase of the MNA level in the blood sample isolated from the patient by at least 20% as compared to the reference level of MNA indicates that the patient will benefit from said therapeutic treatment.

4. The method of claim 1, wherein the reference level is at least 100 nmol/L.

5. A method of determining whether a patient with insulin resistance or type-2 diabetes (T2D) responds to a therapeutic treatment with an inhibitor of nicotinamide-N-methyltransferase (NNMT), said method comprising the steps:
- determining the level of 1-methylnicotinamide (MNA) in a blood sample isolated from the patient; and
- comparing the level of MNA in the blood sample with a pre-treatment level of MNA;
wherein a decrease of the MNA level in the blood sample isolated from the patient as compared to the pre-treatment level of MNA indicates that the patient responds to said therapeutic treatment; and
wherein the patient is a patient to whom at least once an inhibitor of NNMT is administered or has been administered.

6. The method of claim 5, wherein the blood sample is isolated from the patient after an inhibitor of NNMT has been administered to said patient.

7. The method of claim 5 or 6, wherein the pre-treatment level is determined in a blood sample isolated from the patient before an inhibitor of NNMT has been administered to said patient.

8. The method of any one of claims 5 to 7, wherein a decrease of the MNA level by at least 10% in the blood sample isolated from the patient as compared to the pre-treatment level of MNA indicates that the patient responds to said therapeutic treatment.

9. A method of adjusting the dose of a therapeutic drug applied for therapeutic treatment of insulin resistance or type-2 diabetes (T2D) in a patient comprising the steps:
- determining the level of 1-methylnicotinamide (MNA) in a blood sample isolated from the patient; and
- comparing the level of MNA in the blood sample with a pre-treatment level of MNA;
wherein the patient is a patient to whom at least once an inhibitor of NNMT is administered or has been administered; and
wherein said therapeutic drug is an inhibitor of nicotinamide-N-methyltransferase (NNMT).

10. The method of claim 9, wherein the blood sample is isolated from the patient after an inhibitor of NNMT has been administered to said patient.

11. The method of claim 9 or 10, wherein the pre-treatment level is determined in a blood sample isolated from the patient before an inhibitor of NNMT has been administered to said patient.

12. The method of any one of claims 9 to 11, wherein a decrease of the level of MNA in the blood sample when compared to the pre-treatment level of MNA indicates that the dose of the therapeutic drug applied for treating T2D in the patient is sufficient and can optionally be (further) lowered.

13. The method of any one of claims 9 to 11, wherein a lack of decrease of the level of MNA in the blood sample when compared to the pre-treatment level of MNA indicates that the dose of the therapeutic drug applied for treating T2D in the patient is not sufficient and can optionally be increased (again).

14. The method of any one of claims 1 to 13, wherein the inhibitor of NNMT is selected from the group consisting of small molecules, peptides, nucleic acids, and antibodies.

15. A method for determining the degree of insulin resistance in a patient, comprising the steps:
- determining the level of MNA in a blood sample isolated from the patient; and
- comparing the level of MNA in the blood sample with a reference level of MNA;
wherein the degree of the increase of the MNA level in the blood sample isolated from the patient as compared to the reference level of MNA is indicative for the degree of insulin resistance in the patient.

16. The method of claim 15, wherein the reference level is an average MNA level determined from at least two reference samples from healthy subjects.

17. The method of claim 15, wherein the reference level is at least 100 nmol/L.

18. The method of any one of claims 1 to 17, wherein the patient is a mammal, preferably a human, a non-human primate or a rodent.

19. The method of any one of claims 1 to 18, wherein the patient is a human and has a body mass index (BMI) of 25 kg/m² or more.

20. The method of any one of claims 1 to 19, wherein the level of MNA in the blood sample is determined by mass spectrometry.

21. The method of any one of claims 1 to 20, wherein the blood sample is a plasma sample or a serum sample.
